Europäisches Patentamt

European Patent Office

Office européen des brevets

(11) Veröffentlichungsnummer: **0 285 116**
**A2**

(12) **EUROPÄISCHE PATENTANMELDUNG**

(21) Anmeldenummer: 88105134.6

(51) Int. Cl.4: **A61L 2/24** , A61L 2/26 ,
C12Q 1/22

(22) Anmeldetag: 30.03.88

(30) Priorität: 01.04.87 DE 3710848

(43) Veröffentlichungstag der Anmeldung:
05.10.88 Patentblatt 88/40

(84) Benannte Vertragsstaaten:
AT BE CH DE ES FR GB IT LI NL SE

(71) Anmelder: Sartorius GmbH.
Weender Landstrasse 94-108
D-3400 Göttingen(DE)

(72) Erfinder: Sonzogni, Giovanni B.
Via Ronco Alto 15
IT-24018 Villa D'Alme Bergamo(IT)
Erfinder: Thormann, Detlef
Dresdnerstrasse 11
D-3406 Bovenden(DE)
Erfinder: Pradel, Günter
Minkowskiweg 30
D-3400 Göttingen(DE)
Erfinder: Reulecke, Fritz
Burkhardtstrasse 18
D-3404 Adelebsen(DE)
Erfinder: Bosse, Klaus
Uslarerstrasse 12A
D-3417 Bodenfelde(DE)

(74) Vertreter: Köhler, Rudolf
c/o Sartorius GmbH Weender Landstrasse
94-108
D-3400 Göttingen(DE)

(54) Vorrichtung und Verfahren zur Sterilitätsprüfung von Flüssigkeiten und von in eine Flüssigphase überführten Stoffen nach der Membranfiltermethode.

(57) Bei einer Vorrichtung zur Sterilitätsprüfung von Flüssigkeiten und von in eine Flüssigphase überführten Stoffen mittels einer verschließbaren Filterbüchse, aufweisend ein Büchsenunterteil (1) mit drainierender Filterunterstützung (2), Auslaßstutzen (3) mit aufsteckbarer Verschlußkappe (4), einen auf der Filterunterstützung (2) aufliegenden Membranfilter (5), dessen Rand zwischen Büchsenunterteil (1) und einem glockenförmigen transparenten Büchsenoberteil (6) mit Einlaßstutzen (7) und aufsteckbarer Verschlußkappe (8) derart leckdicht zwischen den verbundenen Büchsenteilen (1 und 6) eingeklemmt ist, daß Flüssigkeit vom Büchsenoberteil (6) in das Büchsenunterteil (1) nur durch die Filtermembran (5) gelangen kann, sind mindestens zwei vorstehend spezifizierte Filterbüchsen (B1,B2) und ein spritzenförmiger Trichter (20) mit Kanüle (22,22') am Trichterauslaß zu einem maschinell durch ein Handlings-Gerät (R) bedienbaren Ensemble zusammengestellt. Dieses kann durch einen Büchsenhalter (9) verbunden sein, welcher die Verschlußkappen (4,8) und Stutzen (3,7) der Filterbüchsen (B1 und B2) für Bedienungselemente (29) des Handlings-Gerätes (R) frei zugänglich läßt, Aufnahmelager (14) für Greifer des Handlings-Gerätes aufweist, und eine das Vereinzeln der Filterbüchsen (B1,B2) für eine individuelle Weiterbehandlung erlaubende Büchsenlager (12) aufweist.

Die Vorrichtung ermöglicht eine vollständig automatische Durchführung des Sterilitätstests mit stark verminderter Sekundärkontaminationsgefahr durch den Wegfall von manuellen Eingriffen durch Personal.

**Fig. 4**

### Vorrichtung und Verfahren zur Sterilitätsprüfung von Flüssigkeiten und von in eine Flüssigphase überführten Stoffen nach der Membranfiltermethode

Die Erfindung betrifft eine Vorrichtung und ein Verfahren zur Sterilitätsprüfung von Parenteralia, lyophilisierten oder anderen löslichen Pulvern, Ölen und Salben, gemäß Oberbegriff des Hauptanspruches. Pharmazeutische Produkte, die intravenös, subkutan oder auf offene Wunden appliziert, und Schlauchsysteme, Nadeln usw., die für die Verabreichung verwendet werden, müssen steril sein. Stichprobenartig muß deshalb die Sterilität solcher Produkte kontrolliert werden. Die einzelnen Pharmakopöen wie USP, EP u.a. empfehlen hierfür die Membranfiltrationsmethode anstelle des Direkt-Beimpfungsverfahrens. Gemäß den geltenden Vorschriften der "The United States Pharmacopeia" (USP) werden die zu untersuchenden Proben bei Anwendung der sogenannten Membranfiltermethode hälftig auf zwei geschlossene, sterile Filterbüchsen verteilt und filtriert.

In einem zweiten Schritt werden die Membranfilter und das Büchseninnere mit einer sterilen Lösung filtrierend gespült, wenn aus der zu untersuchenden Probe Hemmstoffe für Mikroorganismen ausgespült werden müssen. Anschließend erfolgt in einem dritten Schritt (nach Verschließen der unteren Büchsenöffnung) das Füllen einer ersten Büchse mit einem ersten Nährmedium für die Erfassung primär anaerob wachsender Mikroorganismen und in einem weiteren Schritt das Füllen der zweiten Filterbüchse mit einem zweiten Nährmedium zur Erfassung aerob wachsender Mikroorganismen. Anschließend werden die Filterbüchsen verschlossen und die beiden Nährmedien mit der Filtermembran in situ werden in den beiden Büchsen bei verschiedenen Temperaturen inkubiert.

Unter bestimmten Bedingungen ist es auch empfehlenswert, eine Probe auf drei Filtrationsbüchsen zu verteilen und mit drei verschiedenen Nährmedien zu bebrüten.

Gemäß USP sind bei der gesamten Prozedur der Vorbereitungen, Übertragungen und Weiterbehandlung aseptische Bedingungen einzuhalten, um die Gefahr von Sekundärkontaminationen möglichst niedrig zu halten. Die diesbezüglichen Arbeiten werden daher in der Regel in sogenannten Reinräumen bzw. reinen Werkbänken durchgeführt.

Durch den nachstehend aufgeführten Stand der Technik "Mitteilung der österreichischen Sanitätsverwaltung", Heft 5, 1975, sowie

DE-OS 25 49 835 (US-PS 4,036,698)
DE-OS 34 15 953 (GB-OS 2 140 699)
EP-OS 1 18 601

US-PS 3 295 686
DE-AS 10 12 436
EP-OS 1 86 764 (US-PS 4,652,367)
US-PS 4,292,405

sind die verschiedensten Formen von Filterbüchsen und Übertragungssysteme für derartige Sterilitätsprüfungen bekannt.

Diese bekannten Vorrichtungen sind für die manuelle Bedienung konzipiert und lassen sich nicht ohne weiteres in eine maschinelle Bedienbarkeit durch sogenannte Handlings-Geräte (Roboter) umfunktionieren.

Seitens der Pharmaindustrie besteht jedoch ein Bedürfnis daran, immer wiederkehrende Vorgänge zu automatisieren und maschinell durchführen zu lassen ("Pharmaceutical quality control using laboratory robotics", Janet R. Strimaitis, INTERNATIONAL LABORATORY, November 1986). Die häufigste Kontaminationsquelle ist bei diesen Arbeiten der Mensch. Durch automatische, maschinelle Abfolge dieser Verfahrensschritte ließe sich die Kontaminationsgefahr verringern.

Es ist allerdings schon bekannt, ein Sterilitätstestsystem aus geschlossenen Büchsen mit Schlauchsystem und Übertragungskanüle, welches für den normalen Handbetrieb mit maschinellen Hilfsmitteln konzipiert ist, in ein Robotersystem zu integrieren ("A robotic system for the sterility testing of injectables", Barbara J. Zlotnick and Michael L. Franklin, PHARMACEUTICAL TECHNOLOGY, May 1987, Seite 58-64). Durch das integrierte Schlauchsystem ist das Sterilitätstestsystem nicht vollständig durch das Robotersystem zu bedienen, so daß eine Vielzahl von Schritten noch manuell durchgeführt werden müssen.

Der Erfindung liegt daher die Aufgabe zugrunde, mit einfachen Mitteln eine Vorrichtung zur Sterilitätsprüfung zu schaffen, welche aufgrund ihrer Form für die maschinelle Bedienung durch Handlings-Geräte funktionsgerecht zu handhaben ist.

Diese Aufgabe wird erfindungsgemäß durch ein Ensemble von sterilen, maschinell mittels eines Handlings-Gerätes in Betriebsfunktion bringbaren Elementen gelöst, von denen eines ein in Betrieb vertikal ausgerichteter spritzenförmiger Trichter mit an seinem unteren Ende angeordneten Kanüle ist und der an seinem oberen Trichterende mit einem Kupplungsanschluß zur Adaption an eine Gas-und gegebenenfalls Flüssigkeitsquelle des Handlings-Gerätes ausgestattet ist. Weiterhin gehören dazu entsprechend der Anzahl verschiedener Inkubationsmedien zwei oder mehr in Betriebsfunktion vertikal ausgerichtete, im wesentlichen identische Filterbüchsen, welche ein Büchsenunterteil mit drainierender Filterunterstützung und verschließbarem Auslaßstutzen, einen auf der Filterunterstützung aufliegenden hydrophilen Membranfilter aufweisen, dessen Rand zwischen Büchsenunterteil und einem glockenförmigen transparenten Büchsenoberteil mit einem zur Einführung der Kanüle des Trichters durch das Handlingsgerät angepaßten Einlaßstutzen aufweist und bei dem die Membran derart dichtend zwischen den verbundenen Büchsenteilen eingeklemmt ist, daß die Flüssigkeit vom Büchsenoberteil in das Büchsenunterteil nur durch das Membranfilter gelangen kann. Zu dem Ensemble gehören weitere Elemente, die die Öffnungen des Trichters und die Öffnungen der Filterbüchsen bedarfsweise im Lagerzustand, Transportzustand, Betriebszustand und Inkubationszustand verschließende Verschluß-und Schutzkappen gegen Sekundärkontamination bilden.

In einer bevorzugten Ausführungsform besteht der Trichter aus einem Oberteil und einem Unterteil einer Filterbüchse, zwischen deren verbundenen Ränder ein Gas-Sterilfilter in Form einer vorzugsweise hydrophoben Membran leckdicht eingeklemmt und entgegen dem Druckgefälle eines Gasstromes abgestützt ist, wobei das Unterteil außenseitig den Kupplungsanschluß zur Adaption an das Kupplungsstück einer Gasquelle des Handlings-Gerätes bildet.

Durch einen umlaufenden Rahmen kann das Ensemble zu einer maschinell durch ein Handlings-Gerät bedienbaren Einheit verbunden sein, welcher auch zur Ablage der Verschlußkappen dienen kann, wenn diese zwecks Beschickung der Filterbüchsen und des Trichters zeitlich begrenzt zu entfernen sind. Anstelle des umlaufenden Rahmens können die Einzelteile des Ensembles auch durch dem Handlings-Gerät zugeordnete Betriebsmittel und Halter auf den einzelnen Stationen des Handlings-Gerätes positioniert werden. Die Einzelteile des Ensembles sind vorzugsweise aus Kunststoff gebildet und bilden Einwegartikel.

Vorteilhafte Weiterbildungen sind in den Unteransprüchen angegeben.

Ein Ausführungsbeispiel ist in der beiliegenden Zeichnung näher erläutert. Dabei zeigt:

Fig. 1 eine Seitenansicht bzw. vertikalen Teilschnitt durch eine erste Ausführungsform einer Sterilitätstesteinheit,

Fig. 2 eine Draufsicht auf den Rahmen mit Filterbüchsen,

Fig. 3 einen Vertikalschnitt durch einen den Filterbüchsen zugeordneten Trichter zur Übertragung von Lösungen,

Fig. 4 einen Vertikalschnitt durch eine weitere Variante der Sterilitätstesteinheit mit einem vom Rahmen ebenfalls gehaltenen Trichter mit Kanüle und angedeuteten Greiferarmen in verschiedenen Positionen,

Fig. 5 eine Seitenansicht übereinandergestapelter Magazine für Ampullen,

Fig. 6 eine Draufsicht auf ein Magazin für Ampullen in einer ersten Ausführungsform,

Fig. 7 eine Frontansicht auf ein Magazin für Ampullen in einer zweiten Ausführungsform,

Fig. 8 eine Seitenansicht mehrerer übereinandergestapelter Magazine für Flaschen,

Fig. 9 eine Draufsicht auf ein Magazin für Flaschen,

Fig. 10 eine Frontansicht auf ein abgewandeltes Magazin gemäß Fig. 9,

Fig. 11 in schematischer Darstellung ein Handlingsgerät, nachfolgend Roboter genannt, mit betriebsbereiter Sterilitätstesteinheit nach Fig. 1 und 2,

Fig. 12 einen Detailschnitt durch die Pneumatikkupplung für den oder die Trichter,

Fig. 13 einen weiteren Roboter für den Verschluß von Vakuumleitungen und

Fig. 14 einen Detailschnitt durch eine Kupplungsvorrichtung für die Einlaßstutzen der Filterbüchsen, wenn diese mit Druckluft beschickt werden,

Fig. 15 einen Detailschnitt durch eine Kupplungsvorrichtung für die Einlaßstutzen der Filterbüchsen, wenn diese mit Spülflüssigkeit und Nährmedien beschickt werden und

Fig. 16 einen Vetikalschnitt durch eine vereinfachte und bevorzugte Ausführungsform des Ensembles aus Einwegelementen in Verbindung mit Kupplungselementen des Handlings-Gerätes bei der Übertragung einer Prüflösung.

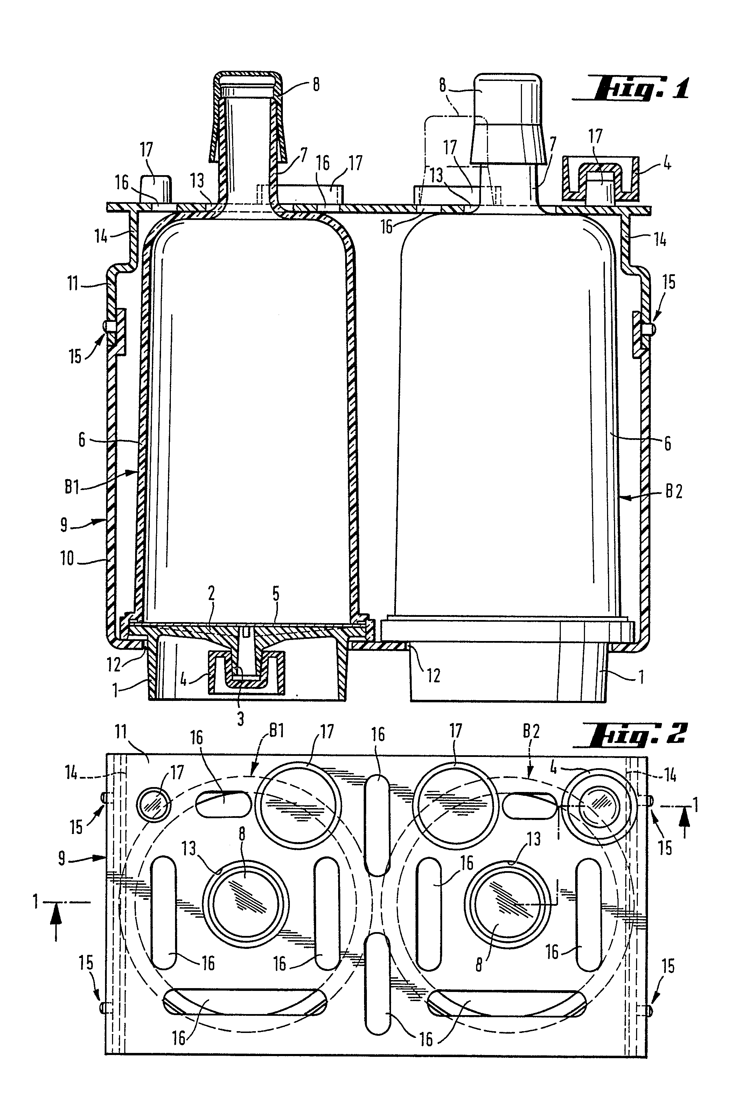

Gemäß Fig. 1 besteht jede der beiden Filterbüchsen B1 und B2 aus einem Büchsenunterteil 1 mit einer drainierenden Filterunterstützung 2, Auslaßstutzen 3, Verschlußkappe 4, einem glockenförmigen Gehäuseoberteil 6, Einlaßstutzen 7 und Verschlußkappe 8. Zwischen Büchsenunterteil 1 und Büchsenoberteil 6 ist randseitig druck-und leckdicht ein Membranfilter 5 mit einer vorgeschriebenen Porengröße von ca. 0,45 $\mu$m eingeklemmt, so daß Flüssigkeit vom Gehäuseoberteil 6 in das Gehäuseunterteil 1 bzw. den Auslaß stutzen 3 nur durch die Filtermembran 5 mittels Druck oder Vakuum hindurch gelangen kann. Die beiden Büchsenteile 6 und 1 sind im gezeigten Ausführungsbeispiel durch Ultraschallschweißung dauerhaft druckdicht miteinander verbunden. Beide Büchsenteile 1 und 6 bestehen aus Kunststoff, wobei das Büchsenoberteil 6 aus transparentem Kunststoff gebildet ist.

Beide Filterbüchsen B1 und B2 sind durch einen gemeinsamen umlaufenden Rahmen 9 zu einer Einheit verbunden. Der Rahmen 9 läuft vertikal und besteht aus einem U-förmigen Rahmenunterteil 10 und einem U-förmigen Rahmenoberteil 11, die mit ihren U-Schenkeln in einer horizontalen Teilungsebene über eine Zapfen-/Loch-Kupplung 15 lösbar miteinander verbunden sind. Die länger gehaltenen U-Schenkel des Rahmenunterteiles 10 lassen sich elastisch federnd gegeneinander bewegen, so daß die Zapfen aus ihren Löchern geführt werden und eine Trennung von Rahmenoberteil 11 und Rahmenunterteil 10 gewährleistet ist. Das Rahmenunterteil 10 hat Durchbrechungen 12, in denen die Büchsenunterteile 1 positioniert sind, wobei auch die Verschlußkappen 4 frei zugänglich sind. Gleichermaßen hat das Rahmenoberteil 11 Durchbrechungen 13, so daß die Verschlußkappen 8 und die Einlaßstutzen 7 der beiden Filterbüchsen B1 und B2 frei zugänglich sind und das Rahmenoberteil 11 auch bei aufgesetzten Verschlußkappen 8 nach oben entfernt werden kann. Die Oberseite des Rahmens 10 weist vier Positionierungslager 17 für die Ablage der Verschlußkappen 4,8 auf, wenn diese für die Beschickung und Entleerung der Filterbüchsen B1 und B2 von den Stutzen 3,7 zeitweise entfernt werden müssen.

Das Rahmenoberteil 11 hat seitlich eingeformte gegenüberliegende Aufnahmelager 14, in welche Greiforgane eines maschinellen Handlings-Gerätes eingreifen können.

Da mit dieser Vorrichtung zur Sterilitätsprüfung in einem Reinraum unter Laminarflow-Bedingungen gearbeitet wird -einem häufig von oben nach unten geführten Luftstrom aus keimfreier Luft - sind zur günstigen Strömungsführung in der Decke des Rahmens 9 mehrere Perforationen 16 angeordnet.

Die in Fig. 1 gezeigte Einheit ist in dieser zusammengesetzten Form sterilisiert und betriebsbereit. Die unteren Verschlußkappen 4 können dabei zweckmäßig bereits vom Hersteller in den beiden dafür vorgesehenen Positionierlager 17 fixiert sein, da für die Durchführung der ersten beiden Schritte über die Auslaßstutzen 3 das Filtrat abgeführt wird. Nach Durchführung der eingangs beschriebenen Operationen und der Befüllung mit Nährmedien ist für die Inkubation der verschiedenen Nährmedien bei unterschiedlicher Temperatur eine Trennung der beiden Filterbüchsen B1 und B2 notwendig. Zu diesem Zweck wird das Rahmenoberteil 11 vom Rahmenunterteil 10 getrennt, so daß beide Filterbüchsen B1 und B2 zur individuellen Einzelbehandlung dem Rahmen 9 entnommen werden können.

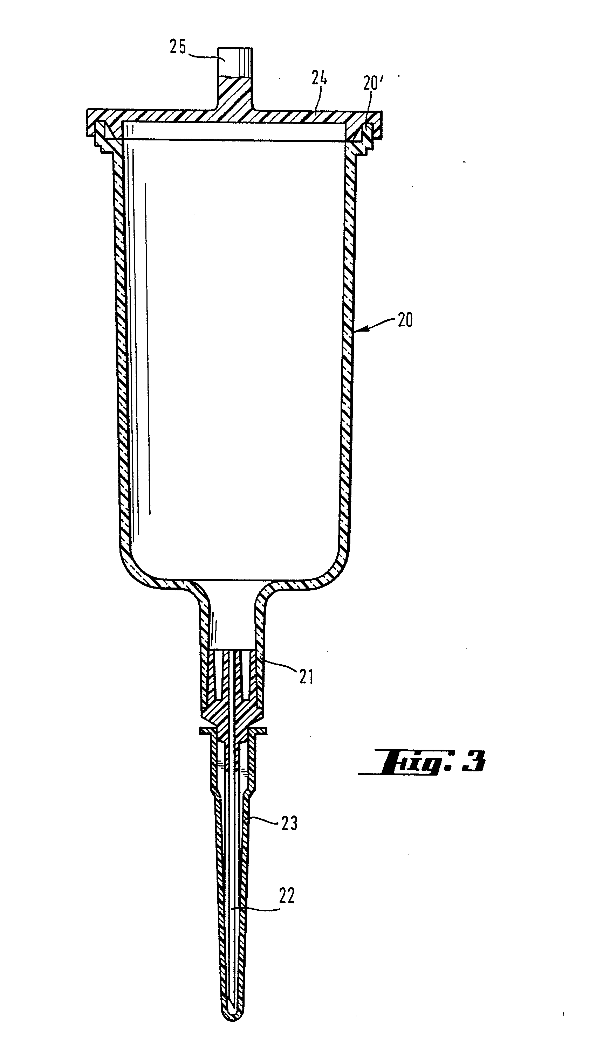

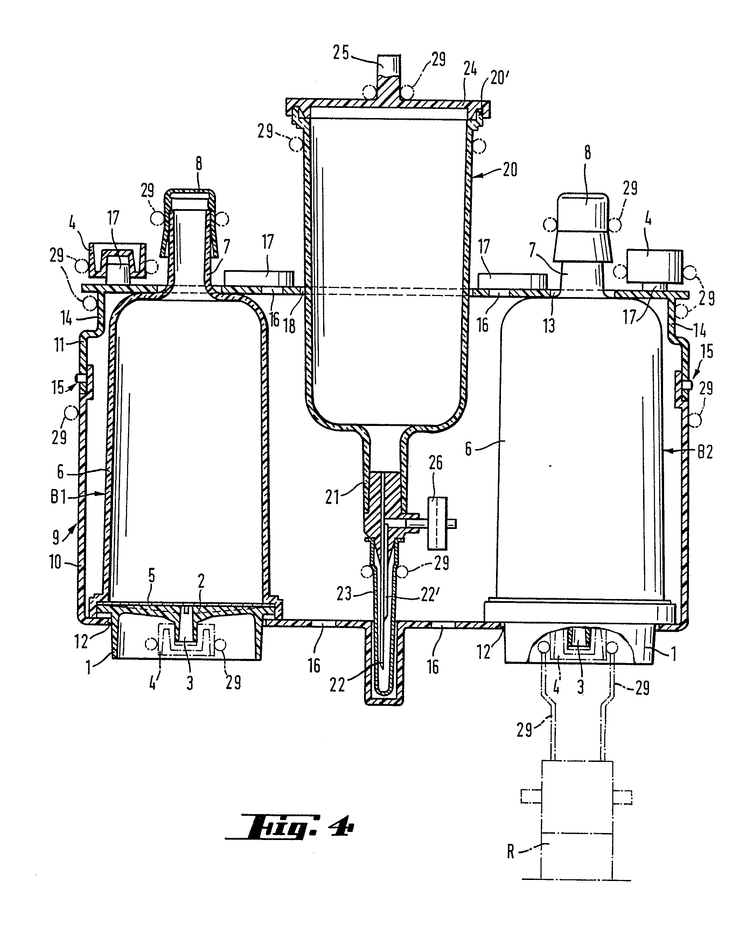

Die in Fig. 1 gezeigte Einheit aus zwei Filtrationsbüchsen B1 und B2 wird gemäß Fig. 3 ergänzt durch mindestens einen ähnlich aufgebauten Trichter 20, dessen Trichterteil im wesentlichen dem Oberteil der Filterbüchsen B1 und B2 entspricht, der einen angeformten Stutzen 21 und eine daran befestigter Kanüle 22 aufweist, so daß eine Art Spritze gebildet ist. Die Kanüle 22 und Stutzen 21 sind durch eine Schutzkappe 23 gegen Kontaminationen gesichert. Gleichermaßen ist die Trichteröffnung durch einen Schutzdeckel 24 mit Deckelgriff 25 gesichert. Der Trichterrand des Trichters 20 hat einen mit einem Handlings-Gerät dichtend kuppelbaren Öffnungsrand.

Der in Fig. 3 dargestellte Trichter 20 ist ebenfalls aus Kunststoff gebildet und ebenfalls sterilisiert und betriebsbereit zusammengebaut.

Die in Fig. 1, 2 und 3 dargestellte Einheit aus Kunststoff gefertigt und für die Einmalverwendung konzipiert. Hierfür sind in erster Linie wirtschaftliche Gründe maßgeblich. Aus wirtschaftlichen Gründen kann es jedoch auch vertretbar sein, das Büchsenoberteil und -unterteil aus autoklavierbeständigem Material zu fertigen und die leckdichte Verbindung zwischen Büchsenoberteil und Büchsenunterteil unter Einklemmung des Filterrandes des Membranfilters 5 unter Zwischenschaltung einer Ringdichtung mit Hilfe einer Überwurfmutter zu verbinden, wie es ebenfalls durch den Stand der Technik bekannt ist. Gleichermaßen kann der Rahmen 9 als wiederverwendbarer Rahmen aus Kunststoff oder Metall gebildet sein, der sich auch autoklavieren läßt.

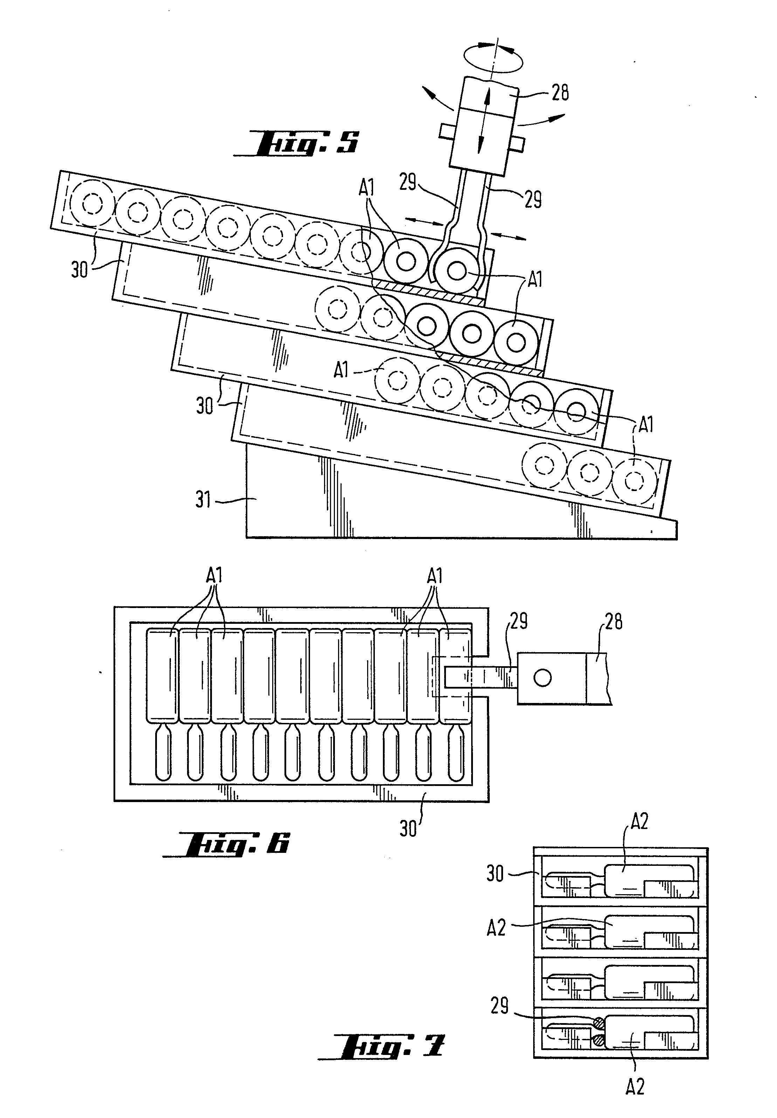

Gemäß Fig. 4 ist in den Rahmen 9 in einer weiteren Durchbrechung 18 der Trichter 20 gemäß Fig. 3 bzw. in modifizierter Ausführungsform positioniert, so daß insgesamt die aus Rahmen 9, den beiden Büchsen B1 und B2 und dem Trichter 20 gebildete Einheit in der dargestellten Ausführungsform betriebsbereit für die Durchführung der Sterilitätsprüfung verschiedener Fluide möglich ist. Der Trichter 20 gemäß Fig. 4 unterscheidet sich von den gemäß Fig. 3 dadurch, daß die Kanülen 22,22' als sogenannte Doppelkanüle ausgebildet sind, wobei die kürzere Kanüle 22' in einem Luftfilter 26 für die Zuführung steriler Luft endet, wenn mit dem Trichter 20 Flüssigkeit aus geschlossenen, unflexiblen Gefäßen entnommen werden soll und ein Nachströmen von steriler Luft in den Behälter notwendig ist.

Bei 29 sind schematisch jeweils die möglichen Positionen der Hände eines Roboters R angedeutet, um die verschiedenen Teile der Einheit erfassen und bewegen zu können.

Gemäß Fig. 4 sind bei der betriebsbereiten Einheit die beiden Verschlußkappen 4 für die Auslaßstutzen 3 bereits in den konischen Positionierlager 17 auf dem Oberteil des Rahmens 9 positioniert, da für die Durchführung des ersten Schrittes bei der Sterilitätsprüfung die Auslaßstutzen 3 der beiden Büchsen B1 und B2 offen sein müssen für den Anschluß an Vakuumleitungen V gemäß Fig. 11,13 und 16. Die angedeutete Position der Hand 29 für die Verschlußkappe 4 am Auslaßstutzen 3 der Büchse B2 zeigt die Verschlußposition, bevor Nährmedium in die einzelnen Büchsen B1,B2 einzufüllen ist.

Die Verschlußkappe 4 einer Filterbüchse B1,B2 kann auch ganz entfallen, wenn statt dessen der Auslaßstutzen 3 bevor Nährmedium in die Filterbüchse eingefüllt wird, durch am Handlings-Gerät R vorgesehene Schweißbacken zugeschweißt wird.

Die Hände 29 der Roboter R sind hier nur - schematisch angedeutet, da diese in verschiedensten Ausführungsformen zum Stand der Technik gehören. Der Roboterarm kann u.a. seine Hände automatisch wechseln, so daß während eines Arbeitsablaufes verschiedene Hände benutzt werden können. Der Roboterarm kann eine Vielzahl von Bewegungen in mehreren Achsen einschließlich Drehbewegungen ausführen, wie dies z.B. durch Roboter der Firma Zymark Corp. 01748 Hopkinton, Mass., USA bekannt ist.

Unter Berücksichtigung dieser Bewegungen und Möglichkeiten wurde die erfindungsgemäße Sterilitätstesteinheit geschaffen.

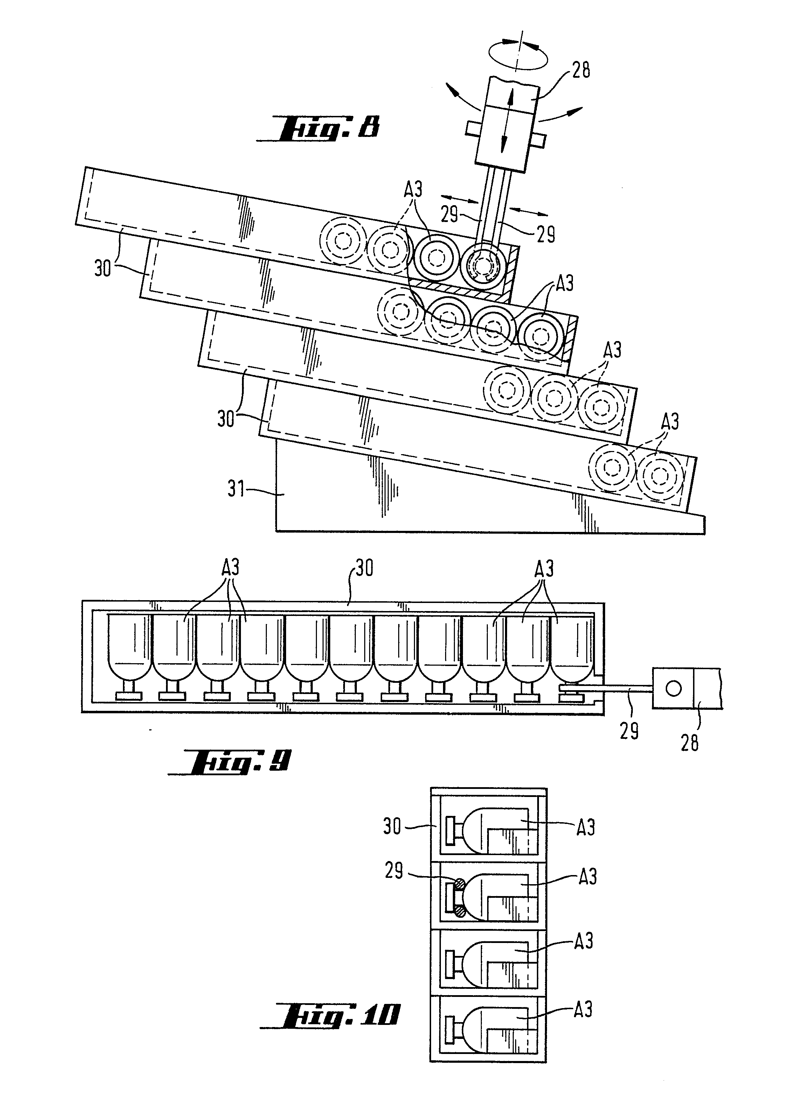

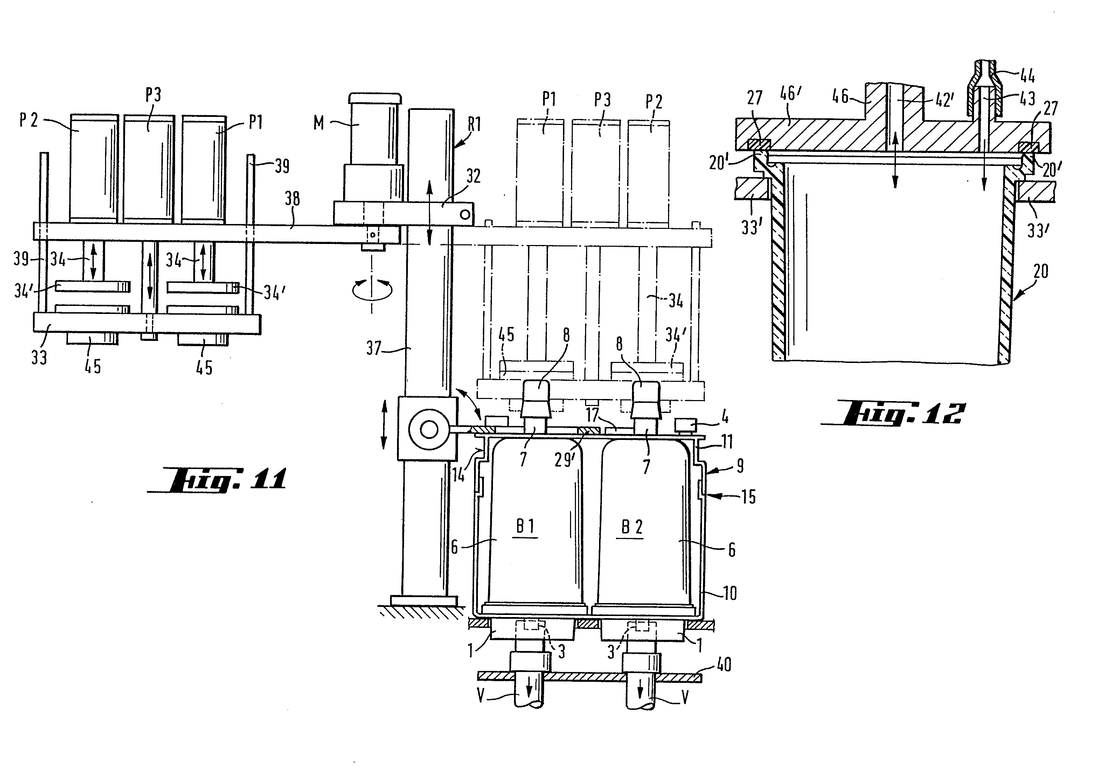

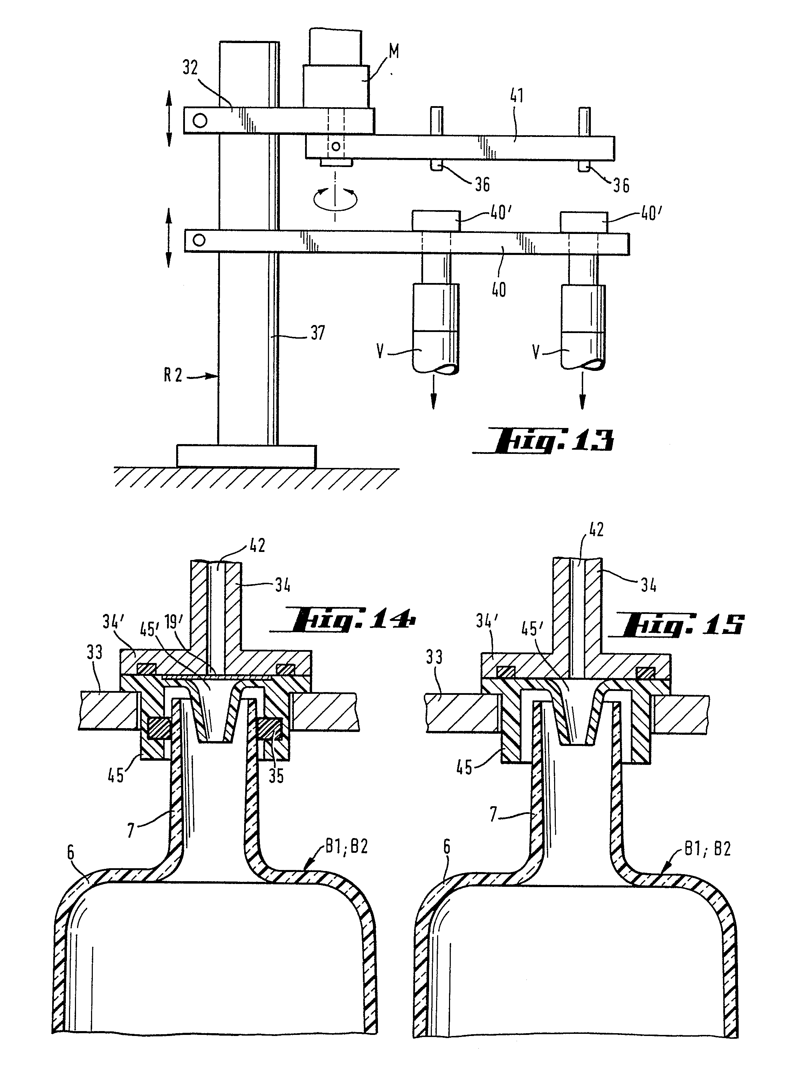

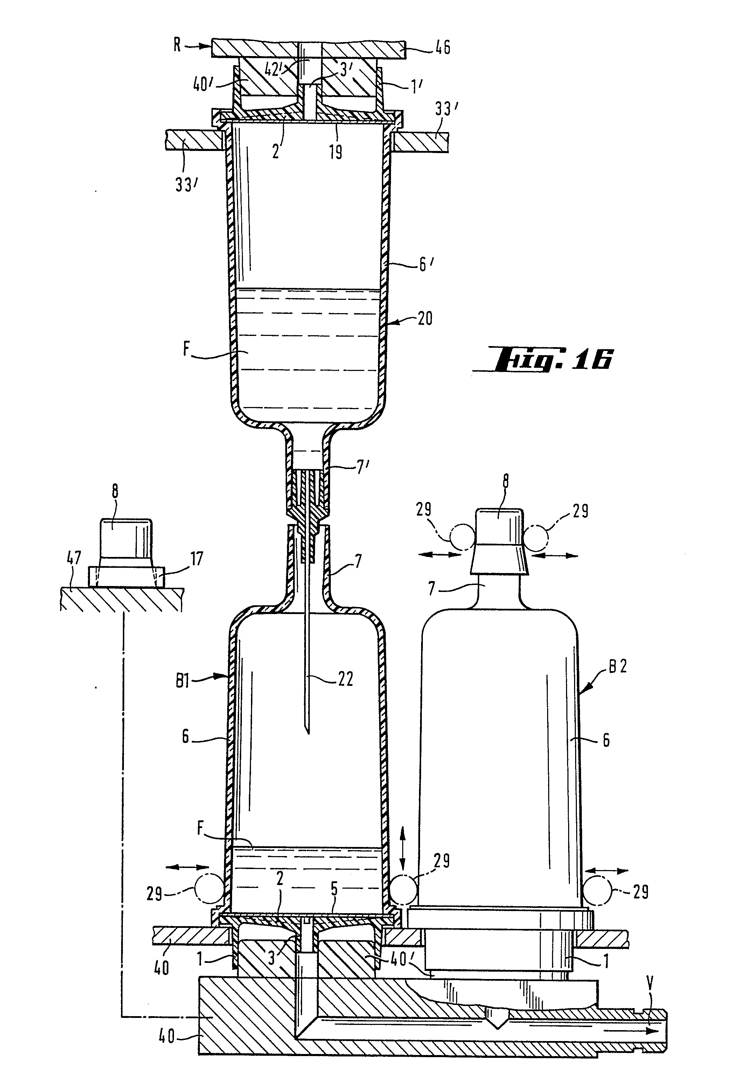

Bei den zu untersuchenden Proben handelt es sich um die eingangs erwähnten Flüssigkeiten oder um die in einer Flüssigphase überführten Stoffe, zumeist Pulver. Diese werden in Ampullen in den verschiedensten Ausführungsformen aus Glas oder Kunststoff abgefüllt. Gemäß Fig. 5 und 6 sind z.B. Ampullen A1 in Magazinen 30 angeordnet. Zur Entnahme der Ampullen A1 sind mehrere Magazine 30 auf einem Magazinhalter 31 übereinandergestapelt, so daß eine Schrägposition zur erleichterten Entnahme der Ampullen A1 durch den Roboterarm 28 bzw. die Hand 29 des Roboters erfolgen kann. Bei den in Fig. 5 bis Fig. 10 gezeigten Magazinen handelt es sich um produktspezifische spezielle Zuführungsmagazine für die Roboter und Teil der Gesamtanlage.

In Fig. 7 sind die Ampullen A2 durch die Hand 29 lediglich an einer anderen günstigen Stelle zu erfassen und vertikal gestapelt.

Ähnlich verhält es sich mit den Flaschen A3 gemäß Fig. 9 und 10, die in verschiedenen Magazinen 30 untergebracht sind, jedoch mit der Hand 29 an den gleichen Stellen erfaßt werden können sowohl bei der schrägen als auch bei der vertikalen Stapelung.

Die Vorrichtung R1 gemäß Fig. 11 in schematischer Darstellung besteht aus einem vertikalen Stativ 37 mit an einem Arm 32 angeordneten Elektromotor M und an einem Schwenkarm 38 gelagerten Pneumatikgeber P1, P2 und P3 sowie einem damit zusammenarbeitenden Kappenhalter 33 für zwei Kappen 45, die in dem Kappenhalter 33 positioniert sind, welcher durch eine Vertikalführung 39 in Richtung auf zwei in Pneumatikgeber P1 und P2 zugeordneten Pneumatikkupplungen 34 bewegbar sind. Die Kupplungsteller 34' bilden gemäß Fig. 14 und 15 mit den Kappen 45 eine Verschlußvorrichtung 34,45, die bedarfsweise mit den Einlaßstutzen 7 der Filterbüchsen B1 und B2 bei abgenommenen Verschlußkappen 8 kuppelbar sind. Durch diese Verschlußvorrichtung 34,45 kann auch die Sekundärkontamination verhindert werden, wenn keine Übertragung von Flüssigkeit in die Filterbüchsen B1 und B2 erfolgt. Dies kann auch dadurch vermieden werden, daß die Einlaßstutzen 7 bei abgenommenen Verschlußkappen 8 durch eine einfache Abdeckplatte der Vorrichtung R1 zugedeckt werden.

Ähnlich aufgebaut ist eine weitere Vorrichtung, die die Verbindung mit dem spritzenförmigen Trichter 20 herstellt. Wie aus Fig. 12 im Detailschnitt ersichtlich ist, wird durch Vertikalbewegung eines Trichterhalters 33' der Oberrand und gleichzeitiger Dichtungsrand 20' des Trichters 20 in Kontakt mit einer Ringdichtung 27 der Pneumatikkupplung 46 gebracht, so daß eine druck-und vakuumdichte Kupplung entsteht. Ein Pneumatikgeber mit steriler Luft kann über die Pneumatikleitung 42' Vakuum oder Druck für die Aufnahme bzw. Abgabe der Lösungen im Trichter 20 erzeugen. Ein mit dem Pneumatikgeber P3 vergleichbarer Pneumatikgeber dient dabei zur Vertikalbewegung des Trichterhalters 33'. Der Teller 46' der Kupplung 46 hat noch mindestens einen weiteren Anschluß 43 für die Zuführung der zu untersuchenden Lösung, wenn Pulver in Flaschen aufgelöst werden muß. Über einen Schlauch mit Peristaltikpumpe wird die Lösung in den Trichter 20 überführt.

Am Stativ 37 ist ein Gegenhalter 29' für den Rahmen 9 an einem gegebenenfalls vertikal verschiebbaren Schwenklager angeordnet. Der Rahmen 9 mit den beiden Filterbüchsen B1 und B2 ist auf einem Leitungshalter 40 für zwei Vakuumleitungen V positioniert, bzw. die Vakuumleitungen V sind mit den Auslaßstutzen 3 der beiden Filterbüchsen B1 und B2 gekuppelt, siehe Fig. 16.

Fig. 13 zeigt eine weitere Vorrichtung R2, welche den Leitungshalter 40 am Stativ 37 aufweist und ebenfalls über einen Motor M mit Schwenkarm 41 verfügt. Mittels des Schwenkarmes 41 zwei Verschlußstopfen 36 oder Kappen lassen sich die Vakuumleitungen V zur Vermeidung von Sekundärkontaminationen absperren bzw. abdecken, wenn keine Filterbüchsen B1 und B2 angekoppelt sind.

Der Trichter 20 muß nicht notwendigerweise das gleiche Volumen haben wie die Filterbüchsen B1 und B2. Im übrigen ist die Darstellung - schematisch vereinfacht.

Die Arbeitsweise der Vorrichtung in einer ersten Methode zur Sterilitätsprüfung in Verbindung mit dem Handlingsgerät wird nachfolgend kurz am Beispiel der Untersuchung für lyophilisierte oder andere lösliche Pulver in Flaschen mit Stopfen beschrieben. Das lyophilisierte Pulver ist z.B. in kleinen Flaschen mit Gummistopfen untergebracht.

Die sterilen Kits gemäß Fig. 1 und 2 sind betriebsbereit in einer Wartestation angeordnet. In gleicher Weise sind die sterilisierten spritzenartigen Trichter 20 mit Schutzkappe 23 und Schutzdeckel 24 gemäß Fig. 3 ebenfalls in einer Wartestation gelagert. Die Flaschen mit dem löslichen Pulver sind in den dafür vorgesehenen Handlingsmagazinen 30 positioniert. Die so betriebsbereite Einrichtung wird gestartet. Ein Roboter benutzt eine dafür ausgebildete Hand 29, um den Schutzdeckel 24 vom Trichter 20 zu entfernen und den Trichter 20 mit seinem Kupplungsrand 20' gemäß Fig. 12 mit der Pneumatikkupplung 46 zu verbinden. In einem gleichzeitigen oder späteren Schritt kann die Schutzkappe 23 von der Kanüle 22 abgestreift werden.

Die beiden Verschlußkappen 8 der beiden Filterbüchsen B1 und B2 werden von den Einlaßstutzen 7 abgenommen und auf dem Rahmen 9 in den Positionierlagern 17 abgesetzt. Diese Einlaßstutzen 7 werden nunmehr gemäß Fig. 11 und 14 durch die Verschlußvorrichtung 34,45 kontaminationssicher verschlossen, wobei je nach Ausbildung der Pneumatikkupplung 34 mehrere Anschlußmöglichkeiten für die verschiedensten Medien wie die Luft, Wasser, Vakuum und verschiedene Reagenzien vorgesehen sind.

Mit Hilfe des gegebenenfalls teleskopartig verlängerbaren Schwenkarmes 38 lassen sich die Kappen 45 mit den Einlaßstutzen 7 der Filterbüchsen B1 und B2 kuppeln. In derselben Station nach Fig. 11 sind die beiden Filterbüchsen B1 und B2 an die Vakuumleitungen V mit ihren unteren Auslässen 3 angeschlossen. Vakuum wird nur angelegt, wenn die Filterbüchsen komplett gefüllt sind.

Ein weiterer Roboter nimmt eine erste Flasche mit Pulver aus einem Magazin und bringt diese zu einem Rüttler. Ein anderer Roboter durchsticht mit Hilfe der Doppelkanüle 22,22' des Trichters 20 den Stopfen der Flasche und füllt über die Schlauchleitung 44 mit Hilfe einer peristaltischen Pumpe sterile Lösung in die Flasche und löst das Pulver. Unter Beibehaltung dieses Kupplungsvorganges erfolgt ein Rütteln der Flasche für einen Zeitraum von ca. 10 bis 15 Sekunden, um das Pulver schneller zu lösen. Durch Anlegen von Vakuum über die Pneumatikleitung 42' der Pneumatikkupplung 46 wird die Flüssigkeit der Flasche und damit das gelöste Pulver in den Trichter 20 gesaugt. Ein Roboter mit einer gabelförmigen Hand streift die leere Flasche mit durchstochenem Stopfen von der Kanüle 22 nach unten in einen Abfallbehälter ab, so daß die Kanüle 22 wieder frei ist.

Mit diesem Inhalt in der Spritze 20 kann durch Wiederholung des Vorganges das Pulver weiterer (4-20 Stück) Flaschen gelöst und entnommen werden. Es ist aber auch möglich, die Flaschen mit Pulver in der zu untersuchenden Anzahl separat in einer Station zu öffnen, durch Zugabe von Flüssigkeit und Rüttelvorgang aufzulösen, die Kanüle einzuführen und erst dann in den Trichter 20 einzusaugen.

Die zu untersuchende Lösung in dem Trichter 20 wird in einem weiteren Schritt bei entfernter Verschlußvorrichtung 34,45 bzw. entfernter Abdeckplatte je zur Hälfte in die beiden Filterbüchsen B1 und B2 eingefüllt und nach Ende des Vorganges werden die Einlaßstutzen 7 wieder durch die Verschlußvorrichtung 34, 45 gemäß Fig. 11 verschlossen. Anschließend wird durch Aktivierung der Vakuumleitungen V filtriert ggf. mit Unterstützung von Druckluft aus den Pneumatikleitungen 42 der Pneumatikzylinder P2 und P1 mit einer Kappe 45 und Dichtungsring 35, die über den Außenkonus des Einlaßstutzens 7 der Filterbüchse B1 bzw. B2 übergreift, Fig. 14. Die dafür vorgesehene (auswechselbare) Kappe 45 weist zweckmäßig einen Gas-Sterilfilter mit 0,22 μm Porengröße auf, der als Membran 19' auf die obere Kappenöffnung 45' aufgesiegelt ist.

In einem weiteren Schritt werden mit Hilfe des Trichters 20 wiederholt sterile Spülflüssigkeit in die Filterbüchsen B1 und B2 eingefüllt und anschließend filtrierend gespült. Alternativ kann dies auch in einer speziellen Spülstation über Kupplungsköpfe nach Fig. 15 (ohne O-Ring-Dichtung im Kappenhals) erfolgen.

Ein Roboter entfernt den Trichter 20 und wechselt gegebenenfalls die Hand um die Filtereinheit mit Rahmen 9 vertikal anzuheben. Ein Roboter nimmt die beiden auf dem Rahmen 9 abgelegten Verschlußkappen 4 für die untere Büchsenöffnung 3 und verschließt diese Büchsenöffnung der beiden Filterbüchsen B1 und B2, wie in Fig. 4 angedeutet oder verschweißt mit einer Schweißzange direkt den Auslaßstutzen 3.

Mit Hilfe einer Vorrichtung werden die beiden Filterbüchsen B1 und B2 mit zwei verschiedenen Nährmedien befüllt. Dies kann ggf. noch über die Kappen 45 und einen (weiteren) Kupplungskopf 34 gemäß Fig. 15 erfolgen. Anschließend werden die beiden Verschlußkappen 8 aus ihren Positionierungslagern 17 am Rahmen 9 entnommen und auf die Einlaßstutzen 7 vorzugsweise dauerhaft durch Rastung aufgesetzt.

Ein Roboter bringt die so präparierte Filtereinheit zu einer Wartestation.

Nachfolgend kann ein neuer Zyklus, wie vorher beschrieben, beginnen.

Zur individuellen Weiterverarbeitung der Filterbüchsen B1 und B2 mit den beiden unterschiedlichen Nährmedien im Brutschrank wird der Rahmen 9 in der Teilungsebene 15 geteilt, d.h. das Rahmenoberteil 11 wird nach oben entfernt und jede Filterbüchse B1 und B2 wird separat aus dem Rahmenunterteil 10 durch einen Greifer 29 entfernt. Für die aerobe Inkubation hat die dafür vorgesehene Filterbüchse, z.B. B2 eine Verschlußkappe 8 mit einem integrierten Luftfilter mit 0,22 μm Porengröße für Sterilbelüftung bzw. einen nach unten gerichteten Belüftungsschlitz.

Um die Sekundärkontamination weitestgehend auszuschließen, sorgen durch die Roboter betätigte Verschlußelemente generell dafür, längere Öffnungszeiten in den einzelnen Übertragungsphasen sowohl bei den Leitungen als auch bei den Kupplungen und Büchsenöffnungen zu vermeiden.

Es versteht sich, daß die Entnahme der Enghalsampullen nach Fig. 6 und 7 bzw. der Enghalsflaschen nach Fig. 9 und 10 sinngemäß erfolgt. Dabei werden die Hälse bzw. Stopfen von einem Roboter abgebrochen bzw. abgeschnitten oder abgedreht und deren Inhalt direkt durch die Kanüle in Trichter 20 eingesaugt. Die übrigen Verfahrensschritte erfolgen sinngemäß nach vorstehender Beschreibung.

Da der Rahmen 9 nach der Ausführung Fig. 1 bis 3 auch entfallen kann, wenn die genaue Positionierung, Weiter beförderung und Fixierung der Einzelelemente durch Betriebsmittel und Halter des Handlings-Gerätes gewährleistet werden kann, ist in Fig. 16 ein erfindungsgemäßes Ensemble ohne verbindenden Rahmen 9 dargestellt, welches sich durch besonders einfache Grundteile auszeichnet, mit denen die Filterbüchsen und der Trichter gebildet sind. Die Spritze 20 besteht dabei aus einem Oberteil 6' einer Filterbüchse B1,B2 und dem Unterteil 1' einer solchen Büchse. Zwischen beiden Büchsenteilen 1' und 6' ist jedoch eine gasdurchlässige Filtermembran 19, vorzugsweise in Form einer hydrophoben Filtermembran von 0,22 μm Porengröße angeordnet, die als Gas-Sterilfilter dient, wenn der Stutzen 3' des Trichters 20 über die Pneumatikleitung 42 mit Druck oder Vakuum beaufschlagt wird. Bei Vakuumbetrieb sorgt die Filterunterstützung 2 für eine Abstützung der Filtermembran. Für die alternative Beaufschlagung mit Druckgas mit geringem Druck reicht eine Verstärkung der Filtermembran mit Vlies oder Gewebe aus. Über den vorhandenen Innenkonus des Büchsenteiles 1' erfolgt über das Kupplungsstück 40' die gasdichte Kupplung zwischen Spritze 20 und der Pneumatikkupplung 46 des Roboters R.

Entsprechend ist der Leitungshalter 40 mit einem Kupplungsstück 40' für die beiden Filterbüchsen B1 und B2 ausgestattet. Diese werden durch die Hand 29 des Roboters R auf die Kupplungsstücke 40' gedrückt und von diesen auch wieder entfernt.

Entsprechend erfolgt die Kupplung des Trichters 20 bzw. der Spritze mit dem Kupplungsstück 40' durch den Trichterhalter 33'. Die Kanüle 22 ist in den Einlaßstutzen 7' des Trichters 20 dichtend eingeklemmt oder eingeklebt. Die Verschlußkappen 8 können auf ein dem Handlings-Gerät bzw. dem Leitungshalter 40 zugeordneten Kappenhalter 47 mit Positionierlager 17 bei geöffneter Filterbüchse abgelegt werden.

Bei der in Fig. 16 dargestellten Flüssigkeit F handelt es sich z.B. um die zu prüfende Lösung, die mittels der Spritze 20 aus einem Prüfbehälter entnommen wird und mittels der Spritze 20 je zur Hälfte in die Filtrationsbüchse B1 und B2 verteilt und filtriert wird.

Bei der Prüfung von hemmstoffhaltigen Substanzen müssen die Filtrationsbüchsen B1 und B2 gespült werden. Die Zufuhr der Spüllösung erfolgt über einen Schwenkarm mit drei Zuführungen von Flüssigkeiten nämlich Spüllösung, Nährlösung 1 und Nährlösung 2. Der Schwenkarm ist mit kurzen Einfüllstutzen 45 ausgestattet, die sich beispielsweise gemäß Fig. 15 mit Spiel (ohne Dichtungsring 35) über die Außenseite des Einlaßstutzens 7 der Filterbüchsen B1 und B2 anlegen. Auf gleiche Weise (mit Dichtungsring 35) ist damit auch die Zufuhr von Druckluft gemäß Fig. 14 möglich, um eine Druckfiltration durchzuführen.

Die einzelnen Verfahrensschritte sind in Anspruch 19 im Ablauf angegeben.

Die Büchsenmagazine und Spritzen-bzw. Trichtermagazine können auch die speziell gestalteten Verpackungen für diese Elemente bilden und eine Entnahme durch Roboter ermöglichen.

Das aus standardisierten Einzelelementen aufgebaute Ensemble gemäß Fig. 16 besteht aus Kunststoffteilen und bildet daher in der Regel preiswerte Einwegartikel, für die sich der Aufwand und das damit verbundene Risiko einer erneuten Sterilisierung nicht lohnt.

Die Anzahl der automatisch zu prüfenden Chargen ohne personelle Betreuung ist abhängig von der Magazingröße, z.B. 20 Chargen mit je 20 Prüflingen. Vorgesehen sind Kontrolleinrichtungen zur Erfassung, ob Prüfbehälter richtig geöffnet sind oder nicht. Weitere Kontrollvorrichtungen stellen sicher, daß der Ablauf störungsfrei erfolgt. Die Chargennummer-Zuordnung erfolgt durch eine vorprogrammierte und automatische Kennzeichnung an den Magazinen oder den Filterbüchsen, z.B. durch Bedruckung.

## Ansprüche

1. Vorrichtung zur Sterilitätsprüfung von Flüssigkeiten und von in eine Flüssigphase überführten Stoffen nach der Membranfiltermethode gemäß Empfehlungen der Pharmakopöen, gekennzeichnet durch ein Ensemble von sterilen, maschinell mittels eines Handlings-Gerätes in Betriebsfunktion bringbaren Elementen, von denen

a) - eines ein im Betrieb vertikal ausgerichteter, spritzenförmiger Trichter (20) mit an seinem unteren Ende angeordneter Kanüle (22) ist und an seinem oberen Trichterende mit einem Kupplungsanschluß (20') zur Adaption an eine Gas- und gegebenenfalls Flüssigkeitsquelle (42,43,46) des Handlings-Gerätes ausgestattet ist,

b) - entsprechend der Anzahl verschiedener Inkubationsmedien zwei oder mehr in Betriebsfunktion vertikal ausgerichtete, im wesentlichen identische Filterbüchsen (B1,B2) sind, aufweisend

b1)-ein Büchsenunterteil (1) mit drainierender Filterunterstützung (2) und verschließbarem Auslaßstutzen (3),

b2)-einen auf der Filterunterstützung (2) aufliegenden hydrophilen Membranfilter (5), dessen Rand zwischen Büchsenunterteil (1) und

b3)-einem glockenförmigentransparenten Büchsenoberteil (6) mit einem zur Einführung der Kanüle (22) des Trichters (20) durch das Handlings-Gerät angepaßten Einlaßstutzen (7), derart dichtend zwischen den verbundenen Büchsenteilen (1,6) eingeklemmt ist, daß Flüssigkeit vom Büchsenoberteil (6) in das Büchsenunterteil (1) nur durch das Membranfilter (5) gelangen kann, und

c) - weitere Elelemente des Ensembles die Öffnungen des Trichters (20) und die Öffnungen der Filterbüchsen (B1,B2) bedarfsweise im Lagerzustand und Betriebszustand verschließende Verschluß-und Schutzkappen (8,4,23,24) gegen Sekundärkontamination sind.

2. Vorrichtung zur Sterilitätsprüfung von Flüssigkeiten und von in eine Flüssigphase überführten Stoffen nach der Membranfiltermethode gemäß Empfehlungen der Pharmakopöen, gekennzeichnet durch ein Ensemble von sterilen, maschinell mittels eines Handlings-Gerätes in Betriebsfunktion bringbaren Elementen, von denen

a) - eines ein im Betrieb vertikal ausgerichteter, spritzenförmiger Trichter (20) mit einer an seinem unteren Ende angeordneten Kanüle (22,22') ist und der an seinem oberen Trichterende mit einem dieses verschließenden Gas-Sterilfilter (19) ausgestattet ist, der in einen Kupplungsanschluß (1',3') zur Adaption an eine Gasquelle (42,46) des Handlings-Gerätes übergeht,

b) - entsprechend der Anzahl verschiedener Inkubationsmedien zwei oder mehr in Betriebsfunktion vertikal ausgerichtete, im wesentlichen identische Filterbüchsen (B1,B2) sind, aufweisend

b1)-ein Büchsenunterteil (1) mit drainierender Filterunterstützung (2) und verschließbarem Auslaßstutzen (3),

b2)-einen auf der Filterunterstützung (2) aufliegenden hydrophilen Membranfilter (5), dessen Rand zwischen Büchsenunterteil (1) und

b3)-einem glockenförmigen transparenten Büchsenoberteil (6) mit einem zur Einführung der Kanüle (22) des Trichters (20) durch das Handlings-Gerät angepaßten Einlaßstutzen (7), derart dichtend zwischen den verbundenen Büchsenteilen (1,6) eingeklemmt ist, daß Flüssigkeit vom Büchsenoberteil (6) in das Büchsenunterteil (1) nur durch das Membranfilter (5) gelangen kann, und

c) - weitere Elemente des Ensembles die Öffnungen des Trichters und die Öffnungen der Filterbüchsen (B1,B2) bedarfsweise im Lagerzustand und Betriebszustand verschließende Verschluß-und Schutzkappen (8,4,23) gegen Sekundärkontamination sind.

3. Vorrichtung nach Anspruch 2, dadurch gekennzeichnet, daß der Gas-Sterilfilter (19) hydrophob ist.

4. Vorrichtung nach Anspruch 1, dadurch gekennzeichnet, daß der Trichter (20) aus dem Oberteil (6') einer Filterbüchse (B1,B2) gebildet ist, in dessen Einlaßstutzen (7',21) die Kanüle (22) integriert ist.

5. Vorrichtung nach Anspruch 2 und 3, dadurch gekennzeichnet, daß der Trichter (20) aus einem Oberteil (6') und einem Unterteil (1') einer Filterbüchse (B1,B2) gebildet ist, zwischen deren verbundenen Ränder der Gas-Sterilfilter (19) in Form einer vorzugsweise hydrophoben Membran leckdicht eingeklemmt und entgegen dem Druckgefälle eines Gasstromes abgestützt ist, wobei das Unterteil (1) außenseitig den Kupplungsanschluß (1',3') zur Adaption an das Kupplungsstück (40') einer Gasquelle (42,46) des Handlings-Gerätes bildet.

6. Vorrichtung nach Anspruch 1 oder 2, dadurch gekennzeichnet, daß ein mindestens zwei vorstehend spezifizierte Filterbüchsen (B1,B2) zu einer maschinell durch ein Handlings-Gerät bedienbare Einheit verbindender Büchsenhalter (9) vorgesehen ist, welcher die Verschlußkappen (4,8) und Stutzen (3,7) der Filterbüchsen (B1 und B2) für Bedienungselemente des Handlings-Gerätes frei zugänglich läßt, Aufnahmelager (14) für Greifer des Handlings-Gerätes aufweist, und das Vereinzeln der Filterbüchsen (B1,B2) für eine individuelle Weiterbehandlung erlaubende Büchsenlager (2) aufweist.

7. Vorrichtung nach Anspruch 6, dadurch gekennzeichnet, daß der Büchsenhalter (9) als Rahmen ausgebildet ist und Positionierlager (17) für die Ablage der von Greifelementen des Handlings-Gerätes erfaßbaren losen Verschlußkappen (4,8) aufweist, vertikal umlaufend ausgebildet ist, eine horizontal verlaufende Teilungsebene (15) aufweist und aus einem oberen Rahmenteil (11) und einem unteren Rahmenteil (10) besteht, die lösbar miteinander verbunden sind.

8. Vorrichtung nach einem der Ansprüche 6 bis 7, dadurch gekennzeichnet, daß der Rahmen (9) aus einem oberen, im wesentlichen rechteckigen U-förmigen Rahmenteil (11) und aus einem unteren, im wesentlichen rechteckigen U-förmigen Rahmenteil (10) gebildet ist und die vier U-Schenkel miteinander durch eine Steckverbindung (15) lösbar verbunden sind.

9. Vorrichtung nach einem der Ansprüche 6 bis 8, dadurch gekennzeichnet, daß das Aufnahmelager (14) für den die Einheit bedienenden Greifer des Handlings-Gerätes an beiden Seiten des oberen Rahmenteiles (11) angeordnet sind und das untere U-förmige Rahmenteil (10) mit den Enden seiner beiden vertikalen Rahmenschenkel federnd nachgiebig in eine Rastverbindung (15) der beiden Schenkelenden des U-förmigen oberen Rahmenteils (11) eingreift.

10.Vorrichtung nach einem der Ansprüche 6 bis 9, dadurch gekennzeichnet, daß das untere Rahmenteil (10) von den Unterteilen (1) der beiden Filterbüchsen (B1,B2) teilweise und das obere Rahmenteil (11) von den Einlaßstutzen (7) mit Verschlußkappen (8) der Filterbüchsen (B1,B2) durchsetzt ist.

11. Vorrichtung nach einem der Ansprüche 6 bis 10, dadurch gekennzeichnet, daß der Rahmen (9) in den horizontalen Rahmenteilen eine vertikale Luftströmung erlaubende Perforation (16) aufweist.

12. Vorrichtung nach Anspruch 1 und 2, dadurch gekennzeichnet, daß das Büchsenoberteil (6) und die übrigen Büchsenteile (8,1,4) aus autoklavierbeständigem Material gebildet sind.

13. Vorrichtung nach einem der Ansprüche 1 bis 11, dadurch gekennzeichnet, daß die Filterbüchsen (B1 und B2) und der Trichter (20) als Einwegartikel aus Kunststoff gebildet sind.

14. Vorrichtung nach Anspruch 1 und 6, dadurch gekennzeichnet, daß der aus Filterbüchsen (B1 und B2) mit Rahmen (9) gebildeten Einheit mindestens ein etwa dem Büchsenoberteil der Filterbüchsen (B1 und B2) angepaßter Trichter (20) mit angeformtem Stutzen (21), daran befestigte Kanüle (22), diese und den Stutzen (23) übergreifender Schutzkappe (23) und einem die Trichteröffnung übergreifenden Schutzdeckel (24) mit einem von einem Handlings-Gerät ergreifbaren Deckelgriff (25) zugeordnet ist, wobei der Trichter (20) zur Übertragung der in die Büchsen (B1 und B2) einfüllbaren Lösungen dient und dessen vom Schutzdeckel (24) befreite Trichteröffnung einen mit einem Handlings-Gerät (34) abdichtend kuppelbaren Öffnungsrand (20') aufweist.

15. Vorrichtung nach Anspruch 1 und 2, dadurch gekennzeichnet, daß der Trichter (20) mit einer Doppelkanüle (22,22') ausgestattet ist, wobei eine Kanüle (22) als Kanüle für Flüssigkeit und eine Kanüle (22') als Kanüle für Luftzufuhr dient und letztere an einem Filter (26) für Sterilluftzufuhr angeschlossen ist.

16. Vorrichtung nach Anspruch 6, dadurch gekennzeichnet, daß der Rahmen (9) eine weitere Durchbrechung (18) für die Aufnahme eines Trichters (20) aufweist, wobei der Trichter (20) für ein Handlingsgerät (29) zugänglich über den Rahmen (18) nach oben hinausragt.

17. Vorrichtung nach Anspruch 1,2 und 6, gekennzeichnet durch deren Verwendung als durch ein maschinelles Handlings-Gerät (R) bedienbares Ensemble von zusammenwirkenden Einzelelementen (9,B1,B2,20) zur Sterilitätsprüfung.

18. Vorrichtung nach Anspruch 1 bis 17, gekennzeichnet durch eine die Vorrichtung zur Sterilitätsprüfung insgesamt sowie in Einzelteilen bewegende, versorgende und entsorgende Handlingsanlage mit programmierter und automatisierter Steuer-und Kontrolleinrichtung.

19. Automatisiertes Verfahren zur Sterilitätsprüfung von Flüssigkeiten und von in eine Flüssigkeitsphase überführten Stoffen nach der Membranfiltermethode unter Verwendung eines Ensemble von Elementen nach Anspruch 1 oder 2 mittels Roboter, dadurch gekennzeichnet, daß

a) in einem ersten Schritt Filterbüchsen (B) in einem Büchsenmagazin, gegebenenfalls in Form ihrer Verpackung, und Spritzen (20) in einem Spritzenmagazin, gegebenenfalls in Form ihrer Verpackung, für die weitere Handhabung durch Roboter (R,29) in einer ersten Station gelagert werden.

b) in einer zweiten Station die Behälter (A1,A2,A3) mit zu untersuchenden Proben in Behältermagazinen (30) gelagert werden,

c) durch Starten eines programmgesteuerten ersten Roboters (R,29),dieser die für die Prüfung einer Charge notwendige Anzahl an Filterbüchsen (B1,B2) zu einer Filtrationsstation bringt und die Büchsenauslässe (3) an eine Absaugvorrichtung (V,40,40') anschließt,

d) dieser erste Roboter anschließend eine Spritze (20) ergreift, diese an eine Pneumatikquelle (42,40') anschließt und die Schutzkappe (23) von der Kanüle (22) streift,

e) ein zweiter programmgesteuerter Roboter (R,29) den ersten Probenbehälter (A1,A2,A3) aus dem Behältermagazin (30) entnimmt und zu einer Öffnungsstation bringt und dieser dort geöffnet wird und

f) falls der Behälter (A1,A2,A3) mit Pulver gefüllt ist, dieses in einer Mischstation mit Flüssigkeit im Behälter (A1,A2,A3) aufgelöst und der Auflösungsprozess gegebenenfalls durch Schütteln in der Mischstation beschleunigt wird,

g) der zweite Roboter (R,29) inzwischen einen weiteren Behälter (A1,A2,A3) aus dem Behältermagazin (30) zur Öffnungsstation bringt und

h) der erste Roboter (R,29) die Spritze (20) in den ersten geöffneten Behälter (A1,A2,A3) mit der zu untersuchenden flüssigen Probe einführt und diese in die Spritze (20) durch Aktivierung der Pneumatikquelle (42,40') eingesaugt wird,

i) der erste Roboter (R,29) den leeren Behälter (A1,A2,A3) an einer Entsorgungsstelle abwirft,

j) der Vorgang nach e) bis i) solange wiederholt wird bis die erforderliche Anzahl von Proben in der Spritze (20) gesammelt ist,

k) der zweite Roboter (R,29) die Verschlußkappen (8) der Filterbüchsen (B1,B2) abnimmt und auf einer Wartestation ablegt,

l) der erste Roboter (R,29) die mit Proben gefüllte Spritze (20) zu der Filtrationsstation bringt und die Probe zu gleichen Teilen auf die Filterbüchsen (B1 und B2) überträgt und nach erfolgter Übertragung die Absaugvorrichtung (V,40,40') aktiviert und die Probe filtriert wird und die Einlässe (7) der Filterbüchsen (B1,B2) abgedeckt werden,

m) am Ende des Prüfvorganges einer Charge der erste Roboter (R,29) die Spritze (20) beseitigt und

n) falls es sich bei den Proben um hemmstoffhaltige Substanzen handelt, die Filterbüchsen (B1,B2) vom ersten Roboter (R,29) von der Absaugvorrichtung (V,40,40') entfernt und zu einer Spülstation mit Spülkopf (33, 45,34',42) für Luft-und Spülmittelzufuhr und Absaugvorrichtung für die Filterbüchsen (B1,B2) versetzt werden
oder der Spülkopf (33,45,34',42) zur Filtrationsstation bewegt wird
und Spüllösung solange in die Filterbüchsen (B1,B2) eingefüllt und anschließend filtriert wird, bis die Hemmstoffe aus den Filterbüchsen (B1,B2) gespült und diese entleert sind,

o) die Filterbüchsen (B1,B2) anschließend zur nächsten Station gebracht werden und der Auslaßstutzen (3) jeder Filterbüchse (B1,B2) durch eine Verschlußkappe (4) oder durch ein Schweißwerkzeug verschlossen wird,

p) jede einzelne Filterbüchse (B1,B2) anschließend auf einer Nährmedienstation mit einem unterschiedlichen Nährmedium beschickt wird,

q) in einer Verschlußstation die Einlaßstutzen (7) der Filterbüchsen (B1,B2) mit den Verschlußkappen (8) verschlossen werden,

r) der erste Roboter (R,29) die so vorbereiteten Filterbüchsen (B1,B2) zu einer weiteren Wartestation bringt und

s) ein neuer Zyklus mit neuen Filterbüchsen (B) in der ersten Station beginnen kann.

Fig. 1

Fig. 2

0 285 116

25

24

20'

20

21

**Fig. 3**

23

22

**Fig. 4**

Fig. 5

Fig. 6

Fig. 7

_Fig. 8_

_Fig. 9_

_Fig. 10_

Fig. 12

Fig. 11

32

M

41

36    36

40'    40'

40

37

R 2

V    V

**Fig.13**

42

34

**Fig.14**

42

34

34'
45'  19'

33

45    35

7

6

B1;B2

34'
45'

33

45

7

6

B1;B2

**Fig.15**

*Fig. 16*